(19) Europäisches Patentamt
European Patent Office
Office européen des brevets

(11) EP 4 019 020 A1

# (12) EUROPEAN PATENT APPLICATION

published in accordance with Art. 153(4) EPC

(43) Date of publication:
**29.06.2022 Bulletin 2022/26**

(21) Application number: **20853834.8**

(22) Date of filing: **19.08.2020**

(51) International Patent Classification (IPC):
***A61K 31/506*** (2006.01) ***A61P 35/02*** (2006.01)
***A61P 35/04*** (2006.01) ***A61P 35/00*** (2006.01)
***A61P 37/06*** (2006.01) ***A61P 19/10*** (2006.01)
***A61P 19/02*** (2006.01) ***A61P 9/00*** (2006.01)
***A61P 3/04*** (2006.01)

(52) Cooperative Patent Classification (CPC):
**A61K 31/506; A61K 45/06; A61P 3/00; A61P 3/04; A61P 3/14; A61P 9/00; A61P 9/10; A61P 11/00; A61P 11/06; A61P 17/00; A61P 17/06; A61P 19/00; A61P 19/02; A61P 19/10; A61P 25/16;** (Cont.)

(86) International application number:
**PCT/CN2020/110061**

(87) International publication number:
**WO 2021/032128 (25.02.2021 Gazette 2021/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.08.2019 CN 201910767562**

(71) Applicants:
- **Hunan Warrant Pharmaceutical Co., Ltd.**
  **Changsha, Hunan 410323 (CN)**
- **Hunan Medical Science and Technology Development Limited Company**
  **Changsha, Hunan 410018 (CN)**
- **HUNAN WARRANT PHARMACEUTICAL TECHNOLOGY DEVELOPMENT CO., LTD**
  **Changsha, Hunan 410330 (CN)**

(72) Inventors:
- **PI, Shiqing**
  **Changsha Hunan 410323 (CN)**
- **XU, Yan**
  **Changsha Hunan 410323 (CN)**
- **YANG, Daihong**
  **Changsha Hunan 410323 (CN)**
- **ZHOU, Zhigang**
  **Changsha Hunan 410323 (CN)**

(74) Representative: **Brand Murray Fuller LLP**
**50 Eastcastle Street**
**London W1W 8EA (GB)**

## (54) APPLICATION OF FLUORO-SUBSTITUTED 2-AMINOTHIAZOLE-5-AROMATIC CARBOXAMIDE

(57) An application, in preparation of medicine for preventing or treating a disease related to a tyrosine kinase, of a compound as represented by formula I, or a pharmaceutically acceptable salt, an ester, a solvate, a prodrug, an active metabolite, a crystal, a stereoisomer, a tautomer, or a geometric isomer thereof, or a pharmaceutical composition comprising the compound as represented by formula I or the pharmaceutically acceptable salt, ester, solvate, prodrug, active metabolite, crystal, stereoisomer, tautomer, or geometric isomer thereof.

EP 4 019 020 A1

(52) Cooperative Patent Classification (CPC): (Cont.)
**A61P 35/00; A61P 35/02; A61P 35/04;**
**A61P 37/00; A61P 37/02; A61P 37/06;**
**A61P 43/00**

## Description

### CROSS REFERENCE TO RELATED APPLICATION

The present application claims the priority and benefit of Chinese patent application No. 201910767562.1 filed with the China National Intellectual Property Administration on August 20, 2019, the disclosure of which is incorporated herein by reference in its entirety.

### TECHNICAL FIELD

The present application pertains to the field of medical chemistry, and particularly is related to a fluoro-substituted 2-aminothiazole-5-aromatic carboxamide for use in the treatment and prophylaxis of diseases and disorders, or use of a pharmaceutical composition thereof in the prophylaxis or treatment of diseases associated with a tyrosine kinase, such as immune diseases, tumors and neurological diseases, etc.

### BACKGROUND

Protein kinases are a class of enzymes that catalyze the phosphorylation of proteins, thereby altering their substrate activity or ability to bind to other proteins. The kinase signaling pathway is the most common form of reversible post-translational modification and controls many aspects of cellular function. Aberrant protein kinase activation is a major hallmark of malignancy, including alterations in cell proliferation, survival, motility and metabolism, as well as diseases such as vascular proliferation and evasion of antitumor immune responses.

1. BCR-ABL

Numerous studies have confirmed the role of Bcr-Abl tyrosine kinase in the pathogenesis of chronic myelogenous leukemia (CML), and Bcr-Abl inhibitors have paved the way for the development of new targeted therapeutic small molecule drugs. Imatinib, Bosutinib, Dasatinib, Nilotinib, Radotinib, and Ponatinib have been approved for the treatment of chronic myelogenous leukemia, eosinophilic syndrome, myeloproliferative/myelodysplastic neoplasms, chronic myelogenous leukemia and acute lymphoblastic leukemia/lymphocytic lymphoma.

Although these drugs have improved the survival rate of cancer patients, they have also caused serious adverse effects, such as severe diarrhea. This problem is particularly present in the stomach and upper gastrointestinal tract where concentrations of drugs are higher after oral administration. At this drug concentration, tyrosinase inhibitors (TKIs) with poor selectivity can inhibit protein kinases that are abundantly expressed in the gastrointestinal tract, resulting in severe toxic reactions which are related to conventional doses.

2. BTK

Bruton's tyrosine kinase (BTK) is a non-receptor kinase that has been demonstrated to be a genetic factor in X-linked agammaglobulinemia. BTK plays a critical role in oncogenic signaling that is important for the proliferation and survival of leukemic cells in many B-cell malignancies. Therefore, BTK is a key target for the development of small molecule inhibitors of B-cell malignancies.

Although Bcr-Abl inhibitors are currently approved for oncology treatment and can radically improve the average survival of patients. However, new genetic mutations and forms of drug resistance also emerge in clinical practice, and further development of new inhibitors is required. High doses of drugs can cause mutations in target proteins, leading to loss of activity. There is still a need to find a low-dose Bcr-Abl inhibitor and reversible BTK inhibitor that can reduce side effects and decrease the likelihood of mutations to meet the medical need.

### SUMMARY

In a first aspect, the present application provides use of a compound of Formula I, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof in the manufacture of a medicament for the prophylaxis or treatment of a disease associated with a tyrosine kinase,

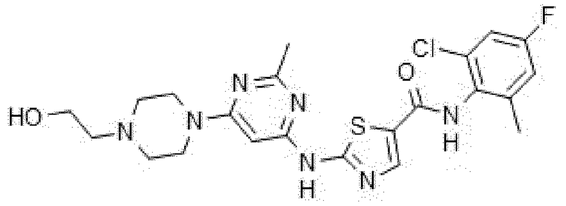

Formula I.

In a second aspect, the present application provides use of the compound of Formula I, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof in the manufacture of a medicament for the inhibition of the activity of a tyrosine kinase.

In a third aspect, the present application provides a method for the prophylaxis or treatment of a disease associated with a tyrosine kinase, comprising administering to a subject in need thereof a therapeutically effective amount of the compound of Formula I, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof.

In a fourth aspect, the present application provides a method for inhibiting the activity of a tyrosine kinase, comprising administering to a subject or a tissue or cells thereof the compound of Formula I, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof. In some embodiments, the method for inhibiting the activity of a tyrosine kinase is performed *in vivo* or *in vitro.*

In a fifth aspect, the present application provides the compound of Formula I, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof for use in the inhibition of the activity of a tyrosine kinase.

In a sixth aspect, the present application provides the compound of Formula I, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof for use in the prophylaxis or treatment of a disease associated with a tyrosine kinase.

In some embodiments of any of the above aspects, the pharmaceutical composition further comprises a pharmaceutically acceptable carrier, excipient or adjuvant.

In some embodiments of any of the above aspects, the medicament further comprises another therapeutic agent for use in combination therapy. In some embodiments, said another therapeutic agent is one or more selected from the group consisting of: cyclophosphamide, isocyclophosphamide, Vincristine, Daunorubicin, Adriamycin, Cytarabine, Mitoxantrone, Dacarbazine, Idarubicin, Tretinoin, Prednisone, Dexamethasone, Mercaptopurine, Methotrexate, Paclitaxel, Melphalan, long-acting interferon, Venetoclax, Crizotinib, Erlotinib, Osimertinib, Ruxolitinib, Afatinib, Erlonat, Imatinib, Lapatinib, Bevacizumab, Trastuzumab, Rituximab, Cetuximab, Blinatumomab, Fludarabine, Gemcitabine, Decitabine, Capecitabine, Bendamustine, Everolimus, Temsirolimus, Etoposide, Adriamycin, Granulocyte Colony Stimulating Factor, Temozolomide, Zoledronic Acid, Oxaliplatin, Cisplatin, Carboplatin and fulvestrant, etc.

In some embodiments of any of the above aspects, said disease associated with a tyrosine kinase is a disease, disorder and condition that benefits from the inhibition or reduction of tyrosine kinase activity.

In some embodiments of any of the above aspects, said tyrosine kinase comprises Bcr-Abl tyrosine kinase and BTK tyrosine kinase.

In some embodiments of any of the above aspects, said disease is selected from cancers.

In some embodiments of any of the above aspects, said cancers are selected from the group consisting of: chronic granulocytic leukemia (CML), gastrointestinal stromal tumor (GIST), small cell lung cancer (SCLC), non-small cell lung cancer (NSCLC), multiple myeloma, solid tumor, B-cell lymphoma, chronic lymphocytic leukemia (CLL), acute lymphocytic leukemia (ALL), non-Hodgkin lymphoma (NHL), small lymphocytic lymphoma (SLL), mantle cell lymphoma (MCL), melanoma, mastocytosis, germ cell tumors, acute myeloid leukemia (AML), marginal/diffuse large B-cell lymphoma, sarcoma, pancreatic cancer, malignant glioma, head and neck tumors, macroglobulinemia, follicular center

lymphoma, prostate cancer, myelodysplastic syndrome, atherosclerotic myeloproliferation, myelofibrosis, eosinophilia, polycythemia vera, liver cancer, advanced sarcoma, glioblastoma multiforme, gliosarcoma, malignant mesothelioma, melanoma, squamous cell carcinoma skin cancer, neuroendocrine tumors, gastric tumors, B-cell acute lymphocytic leukemia, hairy cell leukemia, lymphoplasmacytic lymphoma, follicle center lymphoma, renal cell carcinoma, transitional cell carcinoma, carcinoid tumor, T-cell lymphoma, metastatic non-small cell lung cancer, systemic mastocytosis, metastatic renal cell carcinoma, breast tumor, central nervous system tumor, colorectal neoplasms, metastatic bladder cancer, metastatic pancreatic cancer, metastatic head and neck cancer, ovarian tumor and combinations thereof.

In some embodiments of any of the above aspects, said cancers are selected from cancers resistant to chemotherapeutic agents targeting BCR-ABL and c-KIT, and cancers resistant to Imatinib.

In some embodiments of any of the above aspects, said disease, disorder and condition are selected from the group consisting of: bone metastase, hypercalcemia and/or osteoporosis; pulmonary fibrosis disease; cardiovascular diseases or conditions; mast cell-mediated inflammatory diseases; HTLV-1-associated myelopathy/tropical spastic paralysis; complex regional pain syndrome (CRPS); weight loss or fat loss; artery occlusive disease; ubiquitination; diseases or conditions associated with reduced glucose degradation function; Fridreich's ataxia; Parkinson's disease progressive transplant rejection; rheumatoid arthritis; graft-versus-host disease; autoimmune disease; relapsing immune thrombocytopenic purpura; pemphigus vulgaris; systemic lupus erythematosus; scleroderma pulmonary interstitial fibrosis; and spontaneous urticaria. In some embodiments, said cardiovascular disease or condition is a cardiovascular-like disease caused by RASopathy, or congenital heart disease associated with Noonan or Noonan syndrome.

In some embodiments of any of the above aspects, said mast cell-mediated inflammatory diseases are selected from the group consisting of osteoarthritis, asthma, chronic obstructive pulmonary disease, uveitis, aspirin-exacerbated respiratory disease (AERD), and Parkinson's disease.

## DETAILED DESCRIPTION

### Definition

The following definitions and methods are provided to better define the present application and to guide one of ordinary skill in the art in the practice of the present application. Unless otherwise indicated, terms are understood in accordance with the common usage of one of ordinary skill in the relevant art. All patent references, academic papers, and other published publications cited herein are incorporated herein by reference in their entireties.

The term "optional" or "optionally" means that the subsequently described event or circumstance may or may not occur, and that the description includes instances where said event or circumstance occurs and instances where said event or circumstance does not occur.

The term "pharmaceutically acceptable salt" refers to salts that retain the biological effectiveness of the free acids and bases of the specified compound and that are not biologically or otherwise undesirable. Compounds described herein may possess acidic or basic groups and therefore may react with any of a number of inorganic or organic bases, and inorganic and organic acids, to form a pharmaceutically acceptable salt. These salts can be prepared in situ during the final isolation and purification of the compounds of the present application, or by separately reacting a compound of the present application in its free base form with a suitable organic or inorganic acid, and isolating the salt thus formed. Examples of pharmaceutically acceptable salts include those salts prepared by reaction of the compounds described herein with an inorganic or organic acid, or an inorganic or organic base, such salts include acetate, acrylate, adipate, alginate, aspartate, benzoate, benzenesulfonate, bisulfate, bisulfite, bromide, butyrate, butyn-1,4-dioate, camphorate, camphorsulfonate, caprylate, chlorobenzoate, chloride, citrate, cyclopentanepropionate, decanoate, gluconate, dihydrogenphosphate, dinitrobenzoate, dodecylsulfate, ethanesulfonate, formate, fumarate, glucoheptanoate, glycerophosphate, glycolate, hemisulfate, heptanoate, hexyne-1,6-dioate, hydroxybenzoate, $\gamma$-hydroxybutyrate, hydrochloride, hydrobromide, hydroiodide, 2-hydroxyethanesulfonate, iodide, isobutyrate, lactate, maleate, malonate, methanesulfonate, mandelate, metaphosphate, methoxybenzoate, methylbenzoate, monohydrogenphosphate, 1-napthalenesulfonate, 2-napthalenesulfonate, nicotinate, nitrate, palmoate, pectinate, persulfate, 3-phenylpropionate, phosphate, picrate, pivalate, propionate, pyrosulfate, pyrophosphate, propiolate, phthalate, phenylacetate, phenylbutyrate, propanesulfonate, salicylate, succinate, sulfate, sulfite, suberate, sebacate, sulfonate, tartrate, thiocyanate, tosylate, undeconate and xylenesulfonate. Other acids, such as oxalic acid, while not in themselves pharmaceutically acceptable, may be employed in the preparation of salts useful as intermediates in obtaining the compounds of the present application and their pharmaceutically acceptable acid addition salts (See examples in Berge et al., J. Pharm. Sci. 1977, 66, 1-19). Furthermore, those compounds described herein which may comprise a free acid group may react with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium and aluminum salts and the like. Illustrative examples of bases include sodium hydroxide, potassium hydroxide, choline hydroxide, sodium carbonate, IV'($C_{1-4}$alkyl)$_4$, and the

like. Representative organic amines useful for the formation of base addition salts include ethylamine, diethylamine, ethylenediamine, ethanolamine, diethanolamine, piperazine and the like. It should be understood that the compounds described herein also include the quaternary ammonium compounds of any basic nitrogen-containing groups which they may contain. Water or oil-soluble or dispersible products may be obtained by quaternization. See, for example, the above reference reported by Berge et al.

The term "solvate" refers to a solvate formed from a combination of a compound of the present application with one or more solvent molecules. In some embodiments, the solvate is a monohydrate, e.g., the solvent is water, the monohydrate is formed from a compound of the present application in combination with water.

Esters of the compound of the present invention are those pharmaceutically acceptable esters that are within the scope of sound medical judgment and suitable for use in contact with the patient's tissues without excessive toxicity, irritation, allergic reactions, etc. It should be understood that the esters of the compound of Formula I containing a carboxy or hydroxy group include *in vivo* hydrolysable ester, for example, a pharmaceutically acceptable ester which is hydrolysed in the human or animal body to produce the parent acid or alcohol. Suitable pharmaceutically acceptable esters of carboxyl group formed on the carboxyl group (if present) of the compound of the present invention include, for example, alkyl esters (e.g., $C_{1-6}$ or $C_{1-4}$ alkyl esters), cycloalkyl esters (e.g., $C_{3-12}$, $C_{3-8}$ or $C_{3-6}$ cycloalkyl esters), aryl alkyl esters (e.g., $C_{6-12}$ or $C_{6-8}$ aryl alkyl esters) and heteroaryl alkyl esters (e.g., $C_{6-12}$ or $C_{6-8}$ heteroaryl alkyl esters), etc.

An *in vivo* hydrolyzable ester of the compound of the present invention containing a hydroxy group includes inorganic esters such as phosphate esters and [alpha]-acyloxyalkyl ethers and related compounds which as a result of the *in vivo* hydrolysis of the ester breakdown to give the parent hydroxy group. Further suitable *in vivo* hydrolyzable esters include those formed from amino acids. For example, esters formed by the reaction of the hydroxyl group of the compound with the carboxylic acid of an amino acid. Further suitable *in vivo* hydrolyzable esters include phosphoramidate esters and also the compounds of the present invention in which any free hydroxyl group independently forms a phosphoryl or phosphite ester.

Furthermore, the present invention includes all possible crystalline forms, or polymorphs of the compounds of the present invention, either as single polymorphs, or as a mixture of more than one polymorphs in any ratio. The term "polymorph" refers to a compound of the present application in different crystal lattice forms.

The term "prodrug" as used herein include acid derivatives or alcohol derivatives, etc., well known to practitioners of the art. acid derivatives are, for example, esters prepared by reaction of a parent acid with a suitable alcohol, or amides prepared by reaction of a parent acid with a substituted or unsubstituted amine, or anhydrides, or mixed anhydrides, etc.; alcohol derivatives are, for example, selected from alcohol esters, alcohol alkoxylates, alcohol ethers, carboxylic acids, carboxylic acid esters, phosphorylates, and mixtures thereof, etc. Simple aliphatic or aromatic esters, amides and anhydrides derived from an acidic group pendant on the compound of the present invention and phosphorylates on a hydroxy group of the compound of the present application are particular prodrugs.

The term "active metabolite" refers to a biologically active derivative of a compound that is formed when the compound is metabolized.

Some compounds of the present application may have asymmetric carbon atoms (stereocenters) or double bonds. Therefore, racemates, diastereoisomers, enantiomers, geometric isomers and single isomers are included in the scope of the present application.

When the compounds of the present application contain an ethylenic double bond or other geometric asymmetry center, they include the E and Z geometric isomers unless otherwise specified. Likewise, all tautomerism forms are included within the scope of the present application.

The compounds of the present application may exist as specific geometric or stereoisomeric isomers. The present application contemplates that all of these compounds, including tautomers, *cis*- and *trans*-isomers, (-)- and (+)-enantiomers, (R)- and (*S*)-enantiomers, diastereomers, (D)-isomers, (*L*)-isomers, as well as racemic mixtures and other mixtures, such as enantiomer- or diastereoisomer-enriched mixtures, are included within the scope of the present application. Other asymmetric carbon atoms may exist in substituents such as alkyl. All of these isomers and their mixtures are included within the scope of the present application.

Optically active (R)- and (*S*)-isomers and (D)- and (*L*)-isomers can be prepared by chiral synthesis or chiral reagents or other conventional techniques. If an enantiomer of a compound of the present application is wanted, it can be prepared by asymmetric synthesis or the derivatization action with chiral auxiliaries, in which the resulting diastereomer mixtures are isolated, and the auxiliary groups are cleaved to provide the desired pure enantiomer. Alternatively, when a molecule contains a basic functional group (such as amino) or an acidic functional group (such as carboxyl), the molecule is reacted with an appropriate optical active acid or base to form a diastereomer salt, the diastereomer is resoluted by conventional methods known in the art, and then pure enantiomers can be obtained. In addition, the separation of enantiomers and diastereomers is usually realized by chromatography, and the chromatography employs a chiral stationary phase, and optionally is combined with a chemical derivatization method (e.g. a carbamate is generated from an amine).

The present application also embraces isotopically-labeled compounds of the present application which are

identical to those recited herein, but one or more atoms are replaced by an atom having an atomic mass or mass number different from the atomic mass or mass number usually found in nature. Examples of isotopes that can be incorporated into the compounds of the present application include isotopes of hydrogen, carbon, nitrogen, oxygen, phosphorus, sulfur, fluorine, iodine and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$, etc.

Certain isotopically-labeled compounds of the present application (e.g., those labeled with $^{3}H$ and $^{14}C$) are useful in compound and/or substrate tissue distribution assays. Tritiated ($^{3}H$) and carbon-14 (i.e., $^{14}C$) isotopes are particularly preferred due to their ease of preparation and detectability. Positron emitting isotopes such as $^{15}O$, $^{13}N$, $^{11}C$ and $^{18}F$ are useful for positron emission tomography (PET) studies to determine substrate occupancy. Isotopically labeled compounds of the present application can generally be prepared by following procedures analogous to those disclosed in the Schemes and/or in the Examples herein below by replacing a non-isotopically labeled reagent with an isotopically labeled reagent.

Further, substitution with heavier isotopes such as deuterium (i.e., $^{2}H$) may afford certain therapeutic advantages resulting from greater metabolic stability (e.g., increased in vivo half-life or reduced dosage requirements) and hence may be preferred in some circumstances, wherein the deuterium substitution may be partial or complete, and partial deuterium substitution means that at least one hydrogen is substituted with at least one deuterium, and all such forms of the compounds are included within the scope of the present application.

The term "subject," "patient" or "individual" refers to an individual, suffering from a disease, disorder or condition, which includes mammals and non-mammals. Examples of mammals include, but are not limited to, any member of the mammalia class: humans, non-human primates (e.g., chimpanzees and other apes and monkeys); farm animals, such as cattle, horses, sheep, goats, pigs; domestic animals, such as rabbits, dogs, and cats; and laboratory animals, including rodents, such as rats, mice, and guinea pigs, and the like. Examples of non-human mammals include, but are not limited to, birds, fishes, and the like. In one embodiment of the methods and compositions provided herein, said mammal is a human.

The term "treating" or "treatment" means that the compound or formulation of the present application is administrated to ameliorate or eliminate diseases, or one or more symptoms associated with said diseases, and comprises:

(i) inhibiting a disease or condition, *i.e.,* suppressing the development of the disease or condition;
(ii) alleviating a disease or condition, *i.e.,* causing the regression of the disease or condition.

The term "prophylaxis", "preventing" or "prevention" means that the compound or formulation of the present application is administrated to prevent diseases, or one or more symptoms associated with said diseases, and comprises preventing the occurrence of a disease or condition in an individual, particularly when such individuals are susceptible to the disease or the condition, but have not yet been diagnosed as suffering from said disease or condition.

The terms "effective amount", "therapeutically effective amount" and "pharmaceutically effective amount" refer to a sufficient amount of at least one agent or compound being administered which will relieve to some extent one or more of the symptoms of the disease or disorder being treated. The result can be reduction and/or alleviation of the signs, symptoms or causes of a disease, or any other desired alteration of a biological system. For example, an "effective amount" for therapeutic uses is the amount of the composition comprising a compound as disclosed herein required to provide a clinically significant alleviation of a disease. The effective amount in any individual case may be determined by using techniques, such as a dose escalation study.

The term "acceptable" means having no persistent detrimental effect on the general health of the subject being treated.

The term "pharmaceutically acceptable" refers to a material, such as a carrier or diluent, which does not abrogate the biological activity or properties of the compounds of the present application, and is relatively nontoxic, *i.e.,* the material may be administered to an individual without causing undesirable biological effects or interacting in a deleterious manner with any of the components of the composition in which it is contained.

The term "composition" or "pharmaceutical composition" refers to a biologically active compound, optionally mixed with at least one pharmaceutically acceptable chemical component, including but not limited to, carriers, stabilizers, diluents, dispersing agents, suspending agents, thickening agents and/or excipients.

The term "carrier" refers to relatively nontoxic chemical compounds or agents that facilitate the incorporation of a compound into cells or tissues.

The term "pharmaceutically acceptable adjuvant" refers to those adjuvants which do not have a significant irritating effect on an organic entity and do not impair the biological activity and properties of the active compound. Suitable adjuvants are well known to those skilled in the art, for example carbohydrate, wax, water-soluble and/or water-swellable polymers, hydrophilic or hydrophobic material, gelatin, oil, solvent, or water, etc.

The word "comprise" and variations thereof such as "comprises" or "comprising" will be understood in an open, non-exclusive sense,i.e., "including but not limited to".

The pharmaceutical composition disclosed herein can be prepared by combining the compound disclosed herein with a suitable pharmaceutically acceptable adjuvant, and can be formulated, for example, into a solid, semisolid, liquid, or gaseous formulation, such as tablets pills, capsules powders, granules, lozenges, ointments, syrups, emulsions, suspensions, solutions, suppositories, injections, inhalants, gels, microspheres, aerosols, etc.

Typical routes of administration of the compound or the pharmaceutically acceptable salt, ester, solvate, prodrug, active metabolite, crystal, stereoisomer, tautomer or geometric isomer thereof or the pharmaceutical composition thereof disclosed herein include, but are not limited to, oral, rectal, transmucosal, topical, transdermal, inhalation, parenteral, sublingual, intravaginal, intranasal, intraocular, intraperitoneal, intramuscular, subcutaneous, and intravenous administration. Preferred routes of administration are oral administration and injection administration.

The compounds or pharmaceutical compositions of the present invention may be manufactured according to the formulations and used as the following dosage forms: tablets, capsules or elixirs for oral administration; suppositories for rectal administration; sterile solutions, and suspensions for injection administration; patches and subcutaneous deposits for transdermal administration, etc. The injections may be formulated as the following conventional forms: solutions or suspensions, solid formulations suitable to be prepared into solutions or suspensions before injection, or emulsions. Suitable excipients are, e.g. water, saline, glucose, mannitol, lactose, lecithin, albumin, sodium glutamate, cysteine hydrochloride, etc. In addition, if desired, the pharmaceutical composition for injection may comprise a relatively small amount of non-toxic adjuvants, such as wetting agents, pH buffer and the like. If desired, an absorption enhancer (e.g. liposome) may also be used.

Preparations for parenteral administration include aqueous solutions of active compounds in a form soluble in water. Additionally, the suspensions of active compounds may be formulated into suitable oil suspensions for injection. Suitable lipophilic solvents or carriers include fatty oils such as sesame oil, or other organic oils such as soybean oil, grapefruit oil or apricot kernel oil, or synthetic aliphatic esters such as ethyl oleate or triglyceride, or liposome. Aqueous suspensions for injection may include a substance that enhances the viscosity of the suspensions, such as carboxymethylcellulose sodium, sorbitol or dextran. Optionally, the suspensions may include a suitable stabilizer or a reagent that enhances the solubility of the compound, to enable the preparation of solutions with high concentration.

The pharmaceutical composition according to the present application may be manufactured by using a method known in the art, such as conventional mixing method, dissolution method, granulation method, dragee manufacture method, grinding method, emulsification method, lyophilization method and the like.

In some embodiments, the pharmaceutical composition of the present application is in oral form. For oral administration, the pharmaceutical composition may be formulated by mixing an active compound with a pharmaceutically acceptable adjuvant or excipient well-known in the art. Such adjuvant or excipient enables the compound according to the present application to be formulated into tablets, pills, lozenges, dragees, capsules, powders, granules, liquids, syrups, emulsions, gels, slurries, suspensions, and the like, which are used for oral administration to a patient.

A solid pharmaceutical composition suitable for oral administration may be prepared by a conventional mixing, filling or tabletting method. For example, oral compositions in solid form may be obtained by mixing the active compound with a solid adjuvant or excipient, optionally grinding the resulting mixture, if necessary, adding other appropriate adjuvants or excipients, and then processing the mixture into granules to obtain the cores of a tablet or dragee. Appropriate adjuvants or excipients include, but are not limited to, fillers, binders, diluents, disintegrating agents, lubricants, glidants, sweetening agents, flavoring agents, and the like. The medicinal preparations for oral administration may obtained according to the following method: combing the active compound with a solid excipient, optionally milling the resulting mixture, and processing the particle mixture, if desired, to give a tablet or dragee after adding a suitable adjuvant. Suitable excipients are, specifically, a filler such as a sugar including lactose, sucrose, mannitol or sorbitol; a cellulose preparation such as cornstarch, wheaten starch, rice starch, potato starch, gelatin, gum tragacanth, methyl cellulose, hydroxypropyl methyl cellulose, carboxymethyl cellulose sodium, and/or polyvinyl pyrrolidone (PVP). If desired, a disintegrating agent such as crosslinked polyvinylpyrrolidone, agar or alginic acid or alginate such as sodium alginate may be added. Suitable coating for the dragee is made. For this purpose, a concentrated sugar solution may be employed, the solution may optionally include arabic gum, talc, polyvinyl pyrrolidone, carboxyvinyl polymer gel, polyethylene glycol and/or titanium dioxide, a solution of shellac varnish, and a suitable organic solvent or a solvent mixture. A dye or pigment may be added into the tablet or the coating of the dragee in order to identify or characterize the different combinations of the active compound doses. The preparations may be manufactured according to methods well known in the art.

In all of the administration methods of the compounds or compositions described herein, daily dosage may be, for example, 0.001 to 300 mg/kg body weight, such as 0.01 to 300 mg/kg body weight or 10 to 200 mg/kg body weight in a single dose or in divided doses.

The pharmaceutical composition of the present application may also be suitable for parenteral administration, such as a sterile solution, a suspension, an emulsion or a lyophilized product in an appropriate unit dosage form. A suitable excipient such as a filler, a buffering agent or a surfactant can be used.

The present application develops a kinase inhibitor targeting Bcr-Abl and BTK for the prevention or treatment of immune diseases, tumors and neurological diseases, etc.

In one aspect, the present application provides use of a compound of Formula I, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof in the manufacture of a medicament for the prophylaxis or treatment of a disease associated with a tyrosine kinase,

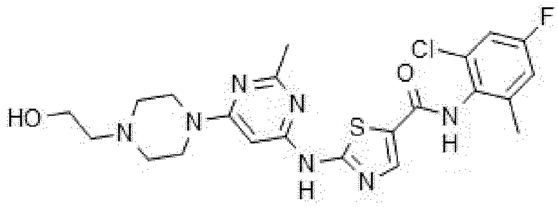

Formula I.

In another aspect, the present application provides use of the compound of Formula I, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof in the manufacture of a medicament for the inhibition of the activity of a tyrosine kinase.

In a further aspect, the present application provides a method for the prophylaxis or treatment of a disease or disorder associated with a tyrosine kinase, comprising administering to a subject in need thereof a therapeutically effective amount of the compound of Formula I disclosed herein, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof.

In still another aspect, the present application provides a method for inhibiting the activity of a tyrosine kinase, comprising administering to a subject or a tissue or cells thereof the compound of Formula I disclosed herein, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof. In some embodiments, the method for inhibiting the activity of a tyrosine kinase is performed *in vivo* or *in vitro*. In some embodiments, the method for inhibiting the activity of a tyrosine kinase comprises an *in vitro* or *in vivo* assay.

In yet another aspect, the present application provides the compound of Formula I disclosed herein, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof for use in the inhibition of the activity of a tyrosine kinase.

In another aspect, the present application provides the compound of Formula I disclosed herein, or a pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof for use in the prophylaxis or treatment of a disease or disorder associated with a tyrosine kinase.

In some embodiments, the pharmaceutical composition disclosed herein further comprises a pharmaceutically acceptable carrier, excipient or adjuvant.

In some embodiments, the medicament disclosed herein further comprises another therapeutic agent for use in combination therapy. In some embodiments, said another therapeutic agent is one or more selected from the group consisting of: cyclophosphamide, isocyclophosphamide, Vincristine, Daunorubicin, Adriamycin, Cytarabine, Mitoxantrone, Dacarbazine, Idarubicin, Tretinoin, Prednisone, Dexamethasone, Mercaptopurine, Methotrexate, Paclitaxel, Melphalan, long-acting interferon, Venetoclax, Crizotinib, Erlotinib, Osimertinib, Ruxolitinib, Afatinib, Erlonat, Imatinib, Lapatinib, Bevacizumab, Trastuzumab, Rituximab, Cetuximab, Blinatumomab, Fludarabine, Gemcitabine, Decitabine, Capecitabine, Bendamustine, Everolimus, Temsirolimus Etoposide, Granulocyte Colony Stimulating Factor, Temozolomide, Zoledronic Acid, Oxaliplatin, Cisplatin, Carboplatin and fulvestrant, etc.

In some embodiments, said disease associated with a tyrosine kinase is a disease, disorder and condition that benefits from the inhibition or reduction of tyrosine kinase activity.

In some embodiments, said tyrosine kinase comprises Bcr-Abl tyrosine kinase and BTK tyrosine kinase.

In some embodiments, said disease is selected from cancers.

In some embodiments, said cancers are selected from the group consisting of: chronic granulocytic leukemia (CML), gastrointestinal stromal tumor (GIST), small cell lung cancer (SCLC), non-small cell lung cancer (NSCLC), multiple myeloma, solid tumor, B-cell lymphoma, chronic lymphocytic leukemia (CLL), acute lymphocytic leukemia (ALL), non-Hodgkin lymphoma (NHL), small lymphocytic lymphoma (SLL), mantle cell lymphoma (MCL), melanoma, mastocytosis, germ cell tumors, acute myeloid leukemia (AML), marginal/diffuse large B-cell lymphoma, sarcoma, pancreatic cancer, malignant glioma, head and neck tumors, macroglobulinemia, follicular center lymphoma, prostate cancer, myelodysplastic syndrome, atherosclerotic myeloproliferation, myelofibrosis, eosinophilia, polycythemia vera, liver cancer, advanced sarcoma, glioblastoma multiforme, gliosarcoma, malignant mesothelioma, melanoma, squamous cell carcinoma skin cancer, neuroendocrine tumors, gastric tumors, B-cell acute lymphocytic leukemia, hairy cell leukemia, lymphoplasmacytic lymphoma, follicle center lymphoma, renal cell carcinoma, transitional cell carcinoma, carcinoid tumor, T-cell lymphoma, metastatic non-small cell lung cancer, systemic mastocytosis, metastatic renal cell carcinoma, breast tumor, central nervous system tumor, colorectal neoplasms, metastatic bladder cancer, metastatic pancreatic cancer, metastatic head and neck cancer, ovarian tumor and combinations thereof.

In some embodiments, said cancers are selected from cancers resistant to chemotherapeutic agents targeting BCR-ABL and c-KIT, and cancers resistant to Imatinib.

In some embodiments, said disease, disorder and condition are selected from the group consisting of: bone metastase, hypercalcemia and/or osteoporosis; pulmonary fibrosis disease; cardiovascular diseases or conditions; mast cell-mediated inflammatory diseases; HTLV-1-associated myelopathy/tropical spastic paralysis; complex regional pain syndrome (CRPS); weight loss or fat loss; artery occlusive disease; ubiquitination; diseases or conditions associated with reduced glucose degradation function; Fridreich's ataxia; Parkinson's disease progressive transplant rejection; rheumatoid arthritis; graft-versus-host disease; autoimmune disease; relapsing immune thrombocytopenic purpura; pemphigus vulgaris; systemic lupus erythematosus; scleroderma pulmonary interstitial fibrosis; and spontaneous urticaria. In some embodiments, said cardiovascular disease or condition is a cardiovascular-like disease caused by RASopathy, or congenital heart disease associated with Noonan or Noonan syndrome.

In some embodiments, said mast cell-mediated inflammatory diseases are selected from the group consisting of osteoarthritis, asthma, chronic obstructive pulmonary disease, uveitis, aspirin-exacerbated respiratory disease (AERD), and Parkinson's disease.

The inventions of the present application provide one or more of the following advantages.

1. The compound of Formula I of the present disclosure have higher kinase inhibitory activity than existing drugs such as Dasatinib.
2. In vitro liver microsomal assays show that the compound of Formula I of the present disclosure has a longer half-life and lower clearance compared to existing drugs such as Dasatinib. The current dosage of Dasatinib is 100 mg per day, the compound of Formula I of the present disclosure is administered at lower dosages and longer intervals than existing drugs such as Dasatinib.

The inhibitor compounds of the present disclosure at a nanomolar concentration (nM) may inhibit ABL1, BTK and BTK (C481S) kinases of the SRC kinase family, and may inhibit the proliferation of leukemic cells in the bone marrow of CML and Ph+ALL by inhibiting the action of these kinases, while normal erythrocytes, leukocytes and platelets can continue to proliferate.

The inventive concepts of the present application improve drug therapeutic effect, reduce dosage and thus reduce toxic side effects, and better provide clinical treatment for immune diseases, tumors, neurological diseases and rheumatoid arthritis, etc. The novel low-dose inhibitors of the present disclosure will provide a broad application prospect for disease treatment.

DETAILED EMBODIMENTS

Specific embodiments of the present invention are described in further detail below in conjunction with examples. The following examples are used for illustrative purposes only and are not intended to limit the scope of the present application.

Preparation Example 1

Synthesis of the compound of Formula I:

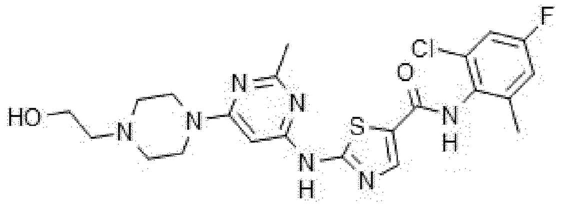

Formula I

The synthesis route of the compound of Formula I is shown as below.

Synthesis of tert-butyl [5-(2-chloro-4-fluoro-6-methylphenylcarbamoyl)thiazol-2-yl] -aminocarboxylate

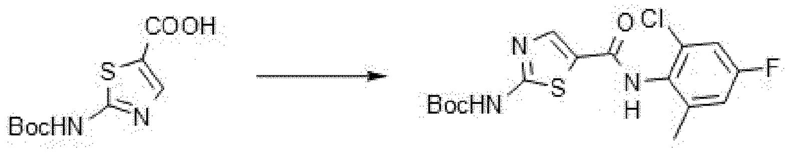

To 250 ml of dichloromethane were added 24.4 g (0.1 mol) of 2-tert-butoxycarbonylaminothiazole-5-carboxylic acid and 0.5 ml of DMF (N,N-dimethylformamide) under protection of $N_2$ gas, and then 13 ml (0.15 mol) of oxalyl chloride solution was added slowly dropwise and reacted for 2 h. The solvent was removed by spin evaporation to obtain a white solid, which was then dissolved in 100 ml of anhydrous dichloromethane. The resulting mixture was slowly added dropwise to a solution of 17.5 g (0.11 mol) of 2-chloro-4-fluoro-6-methylaniline and 38.8 g (0.3 mol) of N,N-diisopropylethylamine in dichloromethane under an ice bath condition, and reacted at room temperature for 10 h under protection of $N_2$ gas. The solvent was removed by distillation under reduced pressure, and to the residue was added a mixed solvent of 25 ml of ethyl acetate and 25 ml of hexane and stirred for 2 h. The resulting mixture was suction-filtered, and the filter cake was eluted with ethyl acetate to give 35.2 g of an off-white powder solid in a yield of 85% and a purity of 95% (ESI-MS (m/z): $[M+H]^+$, 386. $^1H$ NMR(DMSO-$d_6$), $\delta$: 1.49 (s, 9H, $-CH_3$), 2.18 (s, 3H, $-CH_3$), 6.72 (s, 1H, aromatic hydrogen), 6.78 (s 1H, aromatic hydrogen), 8.14 (s, 1H, thiazole hydrogen), 9.51 (s, 1H, -NH), 11.81 (s, 1H, -NH).

Synthesis of 2-amino-N-(2-chloro-4-fluoro-6-methylphenyl)-5-thiazolecarboxamide

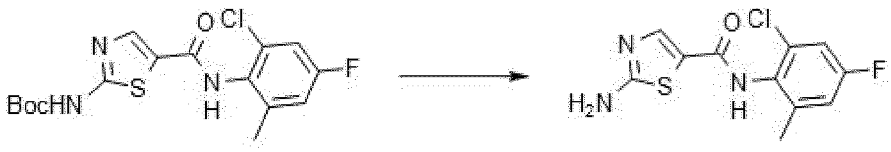

To 200 ml of dichloromethane was added 33.6 g (0.087 mol) of tert-butyl [5-(2-chloro-4-fluoro-6-methylphenylcarbamoyl)thiazol-2-yl]-carbamate, and then 100 ml of trifluoroacetic acid was added and stirred at room temperature for 6 h. TLC detection showed that the reaction was completed, and then the resulting mixture was concentrated under reduced pressure. The resulting oily substance was added into ice water, and the pH was adjusted to 10 with a NaOH solution. The resulting mixture was fully stirred until no oily substance remains, and then the pH was adjusted back to 7. A large amount of solid was precipitated, and then suction-filtered. The filter cake was eluted and then dried to give 23.0 g of a light yellow solid in a yield of 92.5%. ESI-MS (m/z): $[M+H]^+$, 286. $^1H$ NMR(DMSO-$d_6$), $\delta$: 2.21 (s, 3H, $-CH_3$), 6.71 (s, 1H, aromatic hydrogen), 6.76 ( s, 1H, aromatic hydrogen), 7.63 (s, 2H, $-NH_2$), 7.88 (s, 1H, thiazole hydrogen), 9.66 (s, 1H, -NH).

Synthesis of N-(2-chloro-4-fluoro-6-methylphenyl)-2-[(2-methyl-6-chloro-4-pyrimidinyl)amino] - 5 -thiazol ecarboxamide

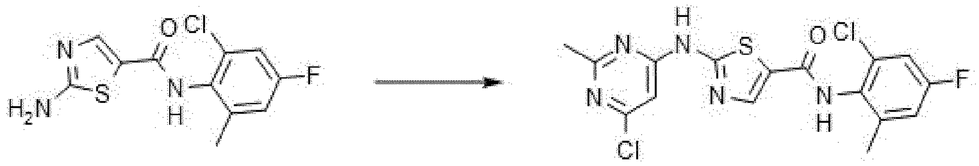

21.3 g (0.075 mol) of 2-amino-N-(2-chloro-4-fluoro-6-methylphenyl)-5-thiazolecarboxamide was dissolved in 100 ml DMF, 17.93 g (0.11 mol) of 2-methyl-4,6-dichloropyrimidine and 48.9 g (0.15 mol) of cesium carbonate were added, and reacted at a temperature maintained at 40°C for 12h. The resulting mixture was suction-filtered. The filtrate was added into 300 ml of ice water, and the pH was adjusted to 6 with diluted hydrochloric acid. Then the resulting mixture was stirred, and a crystal was precipitated and suction-filtered. The filter cake was eluted with ethyl acetate and then dried to give 29.5 g of a light yellow solid in a yield of 95.3%. ESI-MS(m/z): $[M+H]^+$, 412. $^1H$ NMR(DMSO-$d_6$) δ: 2.25 (s, 3H, -$CH_3$), 2.59 (s, 3H, -$CH_3$), 6.98 (s, 1H, pyrimidine hydrogen), 6.73 (s, 1H, aromatic hydrogen), 6.77 (s, 1H, aromatic hydrogen), 8.32 (s, 1H, thiazole hydrogen), 10.01 (s, 1H, -NH), 12.21 (s, 1H, -NH).

Synthesis of N-(2-chloro-4-fluoro-6-methylphenyl)-2-[[6-[4-(2-hydroxyethyl)-1-piperazinyl]-2 -methyl-4-pyrimidinyl]amino]-5-thiazolecarboxamide

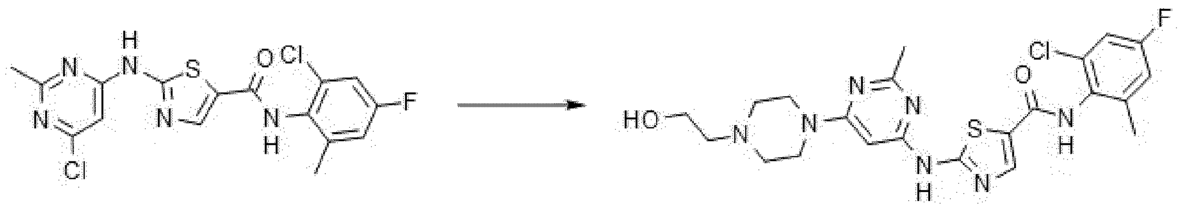

28.2 g (0.068 mol) of N-(2-chloro-4-fluoro-6-methylphenyl)-2-[(2-methyl-6-chloro-4 -pyrimidinyl)amino]-5-thiazolecarboxamide, 44.3 g (0.34 mol) of N-hydroxyethyl piperazine, and 26.4 g (0.204 mol) of N,N-diisopropylethylamine were weighed and dissolved in 250 ml of isopropanol. The resulting solution was warmed to 83°C and reacted under reflux for 8 h. Part of the solvent was removed by concentration under reduced pressure, and the residue was cooled to room temperature and then filtered. The filter cake was recrystallized from a mixed solution of ethanol and water at a ratio of 1:1 to give 31.36 g of a white powder solid in a yield of 91.1% and a purity of 98%. ESI-MS(m/z): $[M+H]^+$, 506. $^1H$ NMR(DMSO-$d_6$), δ: 2.21 (s, 3H, - $CH_3$), 2.41 (s, 3H, -$CH_3$), 2.43 (t, 2H, -$CH_2$), 2.48 (t, 4H, -$CH_2$), 3.52-3.54 (m, 4H, -$CH_2$), 3.55-3.56 (m, 2H, -$CH_2$), 4.48 (s, 1H, - OH), 6.06 (s, 1H, pyrimidine hydrogen), 6.73 ( s, 1H, aromatic hydrogen), 6.77 (s, 1H, aromatic hydrogen), 8.22 (s, 1H, thiazole hydrogen), 9.87 (s, 1H, -NH), 11.45 (s, 1H, -NH).

## Example 1

Studies of in vitro kinase inhibition activity and human liver microsomal metabolism were performed using the compound of Formula I.

1. Kinase inhibition activity study

1.1 Experimental method

1.1.1 Preparation of the test drug

Test group: 50 μl DMSO was used to dissolve the compound of Formula I and Dasatinib, respectively, and the final drug concentration was 10 mM.

Positive control group: staurosporine (from supplier Med Chem) was prepared with DMSO.

1.1.2 Administered Doses

Starting concentration of drugs in the test group: 1 μM
Starting concentration of positive control group: 20 μM
Compound incubation time: 15 min
ATP concentration: 10 μM
Reaction time: 2h

The reaction doses (M) are shown in the following table.

| 10-dose $IC_{50}$(1:4-fold dilution) | 10-dose $IC_{50}$(1:3-fold dilution) |
|---|---|
| Control inhibitor (staurosporine) | drugs of test group |
| 2.00E-05 | 1.00E-06 |
| 5.00E-06 | 3.33E-07 |
| 1.25E-06 | 1.11E-07 |
| 3.13E-07 | 3.70E-08 |
| 7.81E-08 | 1.23E-08 |
| 1.95E-08 | 4.12E-09 |
| 4.88E-09 | 1.37E-09 |
| 1.22E-09 | 4.57E-10 |
| 3.05E-10 | 1.52E-10 |
| 7.63E-11 | 5.08E-11 |

1.1.3 Experimental conditions and procedure

Buffer solution conditions: 20 mM Hepes (pH 7.5), 10 mM $MgCl_2$, 1 mM EGTA, 0.02% Brij35, 0.02 mg/ml BSA, 0.1 mM $Na_3VO_4$, 2 mM DTT, 1% DMSO.

Note: Required enzyme cofactor were added separately to each kinase reaction.

Assay conditions:

| Kinase type | Amount of kinase in RXN (nM) | Supplier |
|---|---|---|
| ABL1 | 0.1 | Invitrogen |
| BTK | 8 | Invitrogen |
| BTK (C481S) | 6 | Signal Chem |

| Substrate | Amount of substrate in RXN | Supplier |
|---|---|---|
| ABLtide | 20μM | GenScript |
| pEY | 0.2mg/ml | Sigma |
| pEY | 0.2mg/ml | Sigma |

Reaction procedure:

a)An indicated substrate was prepared in a freshly made reaction buffer.
b) Any required enzyme cofactors were added into the substrate solution described above.
c) The indicated kinase was added into the substrate solution and gently mixed.
d) The test drugs were added into the kinase reaction mixture using Echo 550.
e) $^{33}$P-ATP (specific radioactivity 0.01μCi/μl) was added into the reaction mixture, and the reaction was initiated.

f) The kinase reaction was incubated at room temperature for 120 min.
g) The reaction was spotted onto P81 ion-exchange paper.
h) The filter was washed with 0.75% phosphoric acid.
i) The radioactive phosphorylated substrate remaining on the filter paper was measured.

1.2 Data analysis

Kinase activity data are expressed as the percent of the remaining kinase activity in the test samples compared with the reactions of the vehicle (dimethyl sulfoxide). $IC_{50}$ values and curve fitting were obtained using Prism4 software (GraphPad).

1.3 Experimental results

| | $IC_{50}$ (nM) | | Ratio | $IC_{50}$ (nM) staurosporine (positive control group) |
|---|---|---|---|---|
| Kinase types | The compound of Formula I | Dasatinib | Dasatinib/the compound of Formula I | |
| ABL1 | 0.0042 | 0.025 | 5.95 | 50.5 |
| BTK | 0.157 | 0.522 | 3.32 | 15.5 |
| BTK (C481S) | 0.032 | 0.113 | 3.53 | 15.7 |

1.4 Experimental conclusion

The inhibitory activities of the compound of Formula I against ABL1, BTK, and BTK(C481S) were significantly higher than that of Dasatinib.

2. Liver microsomal metabolism experiments

2.1 Experimental methods

2.1.1 Incubation conditions

Test compound concentration: 1.0 μM
Buffer solution: 0.05 M phosphate buffer solution (pH 7.4)
Human liver microsomes: Corning® UltraPool™ HLM 150, 20 mg/mL
Microsomal protein concentration: 0.25 mg/mL
NADPH concentration: 1.0 mM
Total reaction volume: 850 μL
Incubation temperature: 37°C
Pre-incubation time: 5 min (no NADPH added)
Sampling time: 0, 5, 10, 15, 20 and 30 min (0, 15 and 30 min for control group)
Sampling volume: 85 μL per time point
Quenching reagent: 85 μL acetonitrile containing 0.25 μM warfarin
Test compounds: Verapamil, Dasatinib, and the compound of Formula I

2.1.2 Experimental design

| Samples | Volume (μL) | | | |
|---|---|---|---|---|
| | Test compounds (100 μM) | Microsomal protein (0.5 mg/mL) | NADPH (2.5 mM) | $PO_4$ buffer solution (50 mM) |
| Control group | 8.5 | 425 | 0 | 416.5 |
| Test group | 8.5 | 425 | 340 | 76.5 |

2.1.3 Experimental procedure

a) Solutions of 10 mM test compounds in DMSO were diluted to 100 μM using acetonitrile: water (50:50).
b) The microsomal proteins were diluted to 0.5 mg/mL with 50 mM phosphate buffer solution and placed in an ice bath (human liver microsomal concentration is 20 mg/ml).
c) 2.5 mM NADPH solution was formulated.
d) The liver microsomal protein, buffer solution, and test compound were pipetted sequentially into 1.5 ml incubation tubes according to the volume in the experimental design table, and incubated at 37°C for 5 min with constant shaking.
e) 85 μL of the quenching reagent was added into a 250 μL quenching vial which was then placed on an ice bath.
f) The reaction was initiated by adding NADPH solution, and 85 μL sample was immediately taken and mixed evenly in the quenching vial with controlled incubation and quenching, this is referred to 0 min sampling point. The sample was capped, vortexed and centrifuged at 4°C.
g) The quenching solution was placed at 4°C for 10 min for the precipitation of the protein.
h) The operations of other sampling points was similar to that of the 0 min sampling point.
i) The sample quenching reaction mixture solution was thoroughly mixed evenly and centrifuged at 14000 rpm for 10 min at room temperature.
j) The supernatant (~100 μl) was pipetted for LC-MS analysis.

2.2 LC-MS analysis method

Instrument: Waters® ACQUITY HPLC-Xevo G2-XS QTof
Chromatographic column: ACQUITYUPLC® BEH $C_{18}$ column 2.1×50 mm, 1.7 μm
Mobile phase: mobile phase A was 0.1% formic acid in water and mobile phase B was 0.1% formic acid in acetonitrile.
Gradient: a gradient of 5 to 95% B in 5.1 min.
Flow rate: 100 μl/min.

The amount of the parent compound at each time point was determined based on the peak area ratio (compound area/warfarin area).

2.3 Calculation equation

The intrinsic clearance (CLint) was calculated by the following equation:

$$\text{Clearance rate constant (k)} = -\text{ slope (1/min)}$$

Half-life ($t_{1/2}$) = 0.693/k
V(μL/mg) = volume of incubation (μL)/ protein amount in the incubation (mg)
Intrinsic clearance (CLint) (μL/min/mg) = V×0.693/$t_{1/2}$

2.4 Experimental results

| Compound | Half-life (min) | Clearance (1/min) | In vitro intrinsic clearance (mL/min/kg) |
|---|---|---|---|
| Dasatinib | 10.9 | 0.0635 | 211 |
| The compound of Formula I | 16.3 | 0.0426 | 142 |
| verapamil (Positive control) | 14.5 | 0.0478 | 159 |

2.5 Experimental Conclusion

*In vitro* liver microsomal assays have shown that the compound of Formula I disclosed herein has a longer half-life and lower clearance compared to Dasatinib, allowing for longer dosing intervals and less frequent of dosing.

Although the present invention has been described above in detail with general description and specific embodiments, it will be apparent to those skilled in the art to make some changes or modifications on the basis of the present invention. Therefore, these changes or modifications made without departing from the spirit of the present invention are within the protection scope of the present invention.

**Claims**

1. Use of a compound of Formula I, or pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof in the manufacture of a medicament for the prophylaxis or treatment of a disease associated with a tyrosine kinase,

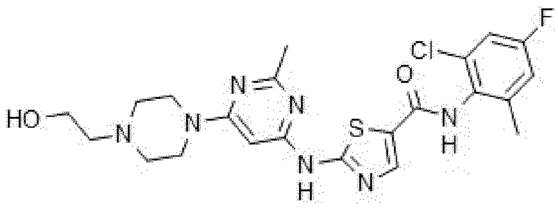

Formula I.

2. Use of a compound of Formula I, or pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof in the manufacture of a medicament for the inhibition of the activity of a tyrosine kinase,

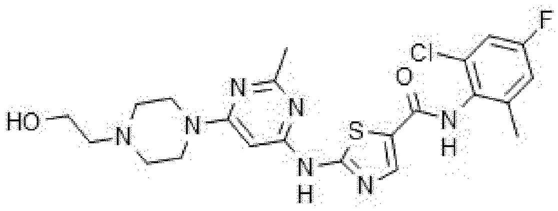

Formula I.

3. A method for the prophylaxis or treatment of a disease associated with a tyrosine kinase, comprising administering to a subject in need thereof a therapeutically effective amount of a compound of Formula I, or pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof,

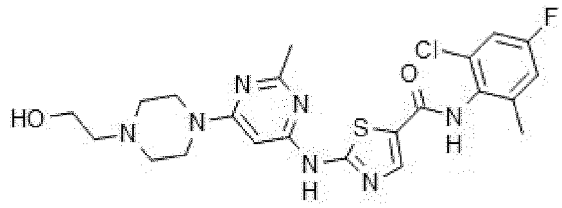

Formula I.

4. A method for inhibiting the activity of a tyrosine kinase, comprising administering to a subject or a tissue or cells thereof a compound of Formula I, or pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof; optionally, said method is performed in vivo or in vitro,

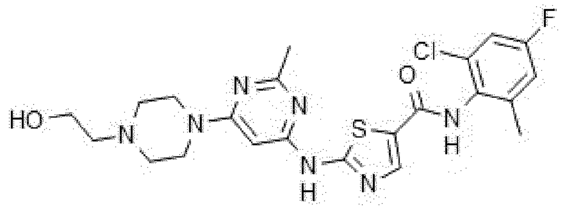

Formula I.

5. A compound of Formula I, or pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof for use in the inhibition of the activity of a tyrosine kinase,

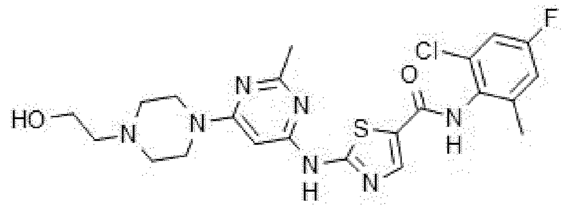

Formula I.

6. A compound of Formula I, or pharmaceutically acceptable salt, ester, solvate, prodrug, active metabolite, crystal, stereoisomer, tautomer or geometric isomer thereof, or a pharmaceutical composition comprising the compound of Formula I, or a pharmaceutically acceptable salt, ester, solvate, prodrug, active metabolite, crystal, stereoisomer, tautomer or geometric isomer thereof for use in the prophylaxis or treatment of a disease associated with a tyrosine kinase,

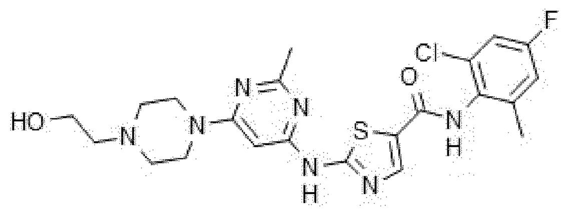

Formula I.

7. The use according to claim 1 or 2, or the method according to claim 3 or 4, or the compound or composition for use

according to claim 5 or 6, wherein the medicament further comprises another therapeutic agent for use in combination therapy;
preferably, said another therapeutic agent is one or more selected from the group consisting of: cyclophosphamide, isocyclophosphamide, Vincristine, Daunorubicin, Adriamycin, Cytarabine, Mitoxantrone, Dacarbazine, Idarubicin, Tretinoin, Prednisone, Dexamethasone, Mercaptopurine, Methotrexate, Paclitaxel, Melphalan, long-acting interferon, Venetoclax, Crizotinib, Erlotinib, Osimertinib, Ruxolitinib, Afatinib, Erlonat, Imatinib, Lapatinib, Bevacizumab, Trastuzumab, Rituximab, Cetuximab, Blinatumomab, Fludarabine, Gemcitabine, Decitabine, Capecitabine, Bendamustine, Everolimus, Temsirolimus Etoposide, Granulocyte Colony Stimulating Factor, Temozolomide, Zoledronic Acid, Oxaliplatin, Cisplatin, Carboplatin and fulvestrant.

**8.** The use according to claim 1 or 7, or the method according to claim 3 or 7, or the compound or composition for use according to claim 6 or 7, wherein:
said disease associated with a tyrosine kinase is a disease, disorder and condition that benefits from the inhibition or reduction of tyrosine kinase activity.

**9.** The use according to any one of claims 1, 2 and 7-8, or the method according to any one of claims 3, 4 and 7-8, or the compound or composition for use according to any one of claims 5, 6 and 7-8, wherein:
said tyrosine kinase comprises Bcr-Abl tyrosine kinase and BTK tyrosine kinase.

**10.** The use according to any one of claims 1 and 7-9, or the method according to any one of claims 3 and 7-9, or the compound or composition for use according to any one of claims 6 and 7-9, wherein said disease is selected from cancers,
preferably, said cancers are selected from the group consisting of: chronic granulocytic leukemia (CML), gastrointestinal stromal tumor (GIST), small cell lung cancer (SCLC), non-small cell lung cancer (NSCLC), multiple myeloma, solid tumor, B-cell lymphoma, chronic lymphocytic leukemia (CLL), acute lymphocytic leukemia (ALL), non-Hodgkin lymphoma (NHL), small lymphocytic lymphoma (SLL), mantle cell lymphoma (MCL), melanoma, mastocytosis, germ cell tumors, acute myeloid leukemia (AML), marginal/diffuse large B-cell lymphoma, sarcoma, pancreatic cancer, malignant glioma, head and neck tumors, macroglobulinemia, follicular center lymphoma, prostate cancer, myelodysplastic syndrome, atherosclerotic myeloproliferation, myelofibrosis, eosinophilia, polycythemia vera, liver cancer, advanced sarcoma, glioblastoma multiforme, gliosarcoma, malignant mesothelioma, melanoma, squamous cell carcinoma skin cancer, neuroendocrine tumors, gastric tumors, B-cell acute lymphocytic leukemia, hairy cell leukemia, lymphoplasmacytic lymphoma, follicle center lymphoma, renal cell carcinoma, transitional cell carcinoma, carcinoid tumor, T-cell lymphoma, metastatic non-small cell lung cancer, systemic mastocytosis, metastatic renal cell carcinoma, breast tumor, central nervous system tumor, colorectal neoplasms, metastatic bladder cancer, metastatic pancreatic cancer, metastatic head and neck cancer, ovarian tumor and combinations thereof.

**11.** The use according to claim 10, or the method according to claim 10, or the compound or composition for use according to claim 10, wherein said cancers are selected from cancers resistant to chemotherapeutic agents targeting BCR-ABL and c-KIT, and cancers resistant to Imatinib.

**12.** The use according to claim 8, or the method according to claim 8, or the compound or composition for use according to claim 8, wherein said disease, disorder and condition are selected from the group consisting of: bone metastase, hypercalcemia and/or osteoporosis; pulmonary fibrosis disease; cardiovascular diseases or conditions; mast cell-mediated inflammatory diseases; HTLV-1-associated myelopathy/tropical spastic paralysis; complex regional pain syndrome (CRPS); weight loss or fat loss; artery occlusive disease; ubiquitination; diseases or conditions associated with reduced glucose degradation function; Fridreich's ataxia; Parkinson's disease progressive transplant rejection; rheumatoid arthritis; graft-versus-host disease; autoimmune disease; relapsing immune thrombocytopenic purpura; pemphigus vulgaris; systemic lupus erythematosus; scleroderma pulmonary interstitial fibrosis; and spontaneous urticaria;

preferably, said cardiovascular disease or condition is a cardiovascular-like disease caused by RASopathy, or congenital heart disease associated with Noonan or Noonan syndrome;
preferably, said mast cell-mediated inflammatory diseases are selected from the group consisting of osteoarthritis, asthma, chronic obstructive pulmonary disease, uveitis, aspirin-exacerbated respiratory disease (AERD), and Parkinson's disease.

## INTERNATIONAL SEARCH REPORT

International application No. **PCT/CN2020/110061**

**A. CLASSIFICATION OF SUBJECT MATTER**

A61K 31/506(2006.01)i; A61P 35/02(2006.01)i; A61P 35/04(2006.01)i; A61P 35/00(2006.01)i; A61P 37/06(2006.01)i; A61P 19/10(2006.01)i; A61P 19/02(2006.01)i; A61P 9/00(2006.01)i; A61P 3/04(2006.01)i

According to International Patent Classification (IPC) or to both national classification and IPC

**B. FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

CNABS; DWPI; SIPOABS; JPABS; CNTXT; WOTXT; EPTXT; USTXT; JPTXT; 中国药物专利数据库; CTCMPD; CNKI; 万方数据知识服务平台; Wanfang Data Knowledge Service Platform; Web of Science; PubMed; STN; 百度学术; BAIDU XUESHU: 1174416-41-1, 达沙替尼, 达萨替尼, 达莎替尼, dasatinib, desatinib, Sprycel, BMS 354825, BMS 35482513, BMS-354825, BMS354825, 000-913-50, 302962-49-8, 类似物, 衍生物, analog, 氟, fluoro, fluorin+, 酪氨酸激酶, tyrosine kinase, TKI, ABL, BTK, 瘤, 癌, 白血病, cancer, tumor, tumour, neoplasm, carcinoma, leukemia, leucocythemia

**C. DOCUMENTS CONSIDERED TO BE RELEVANT**

| Category* | Citation of document, with indication, where appropriate, of the relevant passages | Relevant to claim No. |
|---|---|---|
| X | LI, Xiaohai et al. "Characterization of Dasatinib and its Structural Analogs as CYP3A4 Mechanism-Based Inactivators and the Proposed Bioactivation Pathways." *Drug Metabolism and Disposition.*, Vol. 37, No. 6, 31 December 2009 (2009-12-31), ISSN: 0090-9556, pp. 1242-1250 | 1-12 |
| A | 杨燕 (YANG, Yan). "BRD4和靶向酪氨酸激酶小分子抑制剂的设计、合成及生物活性研究 (Design, Synthesis and Biological Activity of BRD4 and Small-molecule Targeted Tyrosine Kinase Inhibitor)" *中国优秀硕士学位论文全文数据库工程科技I辑 (China Master's Theses Full-text Database, Engineering Science & Technology I),* No. 01, 15 January 2019 (2019-01-15), ISSN: 1674-0246, B016-1468 | 1-12 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 November 2020** | **30 November 2020** |
| Name and mailing address of the ISA/CN<br>**China National Intellectual Property Administration (ISA/CN)<br>No. 6, Xitucheng Road, Jimenqiao Haidian District, Beijing 100088<br>China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/110061**

**C. DOCUMENTS CONSIDERED TO BE RELEVANT**

| Category* | Citation of document, with indication, where appropriate, of the relevant passages | Relevant to claim No. |
|---|---|---|
| A | 刘舒畅等 (LIU, Shuchang et al.). "BCR-ABL蛋白激酶抑制剂的研究进展 (Progress of BCR-ABL Kinase Inhibitors)" *中国医药工业杂志 (Chinese Journal of Pharmaceuticals),* Vol. 41, No. 4, 30 April 2010 (2010-04-30), ISSN: 1001-8255, pp. 293-297 | 1-12 |
| A | 邓媛等 (DENG, Yuan et al.). "依鲁替尼与达沙替尼对急性淋巴细胞白血病细胞增殖的抑制及其机制实验研究 (Effects of PCI-32765 and Dasatinib on the Acute Lymphoblastic Leukemic Cells and Their Mechanisms)" *中国实验血液学杂志 (Journal of Experimental Hematology),* Vol. 25, No. 1, 28 February 2017 (2017-02-28), ISSN: 1009-2137, pp. 72-79 | 1-12 |
| A | CN 106749223 A (GUANGZHOU INSTITUTES OF BIOMEDICINE AND HEALTH, CHINESE ACADEMY OF SCIENCES) 31 May 2017 (2017-05-31) claims 1-10 | 1-12 |

**INTERNATIONAL SEARCH REPORT**

International application No.
**PCT/CN2020/110061**

**Box No. II** **Observations where certain claims were found unsearchable (Continuation of item 2 of first sheet)**

This international search report has not been established in respect of certain claims under Article 17(2)(a) for the following reasons:

1. ☑ Claims Nos.: **3-4，7-12**
because they relate to subject matter not required to be searched by this Authority, namely:

[1] Claims 3, 4 and 7-12 relate to a method of the prevention and treatment of diseases associated with tyrosine kinase, and/or a method of the inhibition of activity of tyrosine kinase, therefore do not warrant an international search according to the criteria set out in Rule 39.1(iv). It is expected that the applicant may modify the subject matter as the corresponding pharmaceutical use. The international search report is made on the basis of the subject matter of reasonably expected amendment.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2020/110061**

| Patent document cited in search report | Publication date (day/month/year) | Patent family member(s) | Publication date (day/month/year) |
|---|---|---|---|
| CN 106749223 A | 31 May 2017 | CN 106749223 B | 20 December 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201910767562


### Non-patent literature cited in the description


- **BERGE et al.** *J. Pharm. Sci.,* 1977, vol. 66, 1-19